# EUROPEAN PATENT SPECIFICATION

(11) **EP 3 454 652 B1**
(45) Date of publication and mention of the grant of the patent: **02.10.2024**
(21) Application number: 17795730.5
(22) Date of filing: 13.05.2017
(51) Int. Cl.: A01N 1/02, C12N 5/00, A61K 9/10, A61K 35/28, A61K 47/36, C12N 5/0775, C12N 5/073

(54) **SOLUTION FOR THE PRESERVATION, TRANSPORT AND APPLICATION OF STEM CELLS**
LÖSUNG ZUR KONSERVIERUNG, ZUM TRANSPORT UND ZUR ANWENDUNG VON STAMMZELLEN
SOLUTION POUR LA CONSERVATION, LE TRANSPORT ET L'APPLICATION DE CELLULES SOUCHES

(30) Priority: 13.05.2016 CZ 20160284
(43) Date of publication of application: 20.03.2019
(73) Proprietor: Institute of Experimental Medicine of the CAS, 14200 Praha 4 (CZ); Bioinova, a.s., 14200 Praha 4 (CZ)
(72) Inventor: PETRENKO, Yuriy, Charkov (UA); SYKOVA, Eva, 16000 Praha 6 (CZ); CEJKOVA, Jitka, 10200 Praha 10 (CZ); VACKOVA, Irena, 10400 Praha 22 (CZ); GROH, Tomas, 40002 Usti nad Labem (CZ)
(74) Representative: Kratochvil, Vaclav
(86) International application number: PCT/IB2017/052832
(87) International publication number: WO 2017/195179

(56) References cited:
- EP-A1- 0 580 444
- EP-A1- 1 647 186
- EP-A1- 2 639 296
- WO-A1-2009/120996
- US-A1- 2007 026 377
- BUCHANAN S S ET AL: "Cryopreservation of Stem Cells Using Trehalose: Evaluation of the Method Using a Human Hematopoietic Cell Line", STEM CELLS AND DEVELOPMENT, MARY ANN LIEBERT, INC. PUBLISHERS, US, vol. 13, no. 3, 1 June 2004 (2004-06-01), pages 295 - 305, XP008120063, ISSN: 1547-3287

## Description

### FIELD OF THE INVENTION

The invention applies to a solution for the preservation, transport and application of stem cells for therapeutic use, which consists of a buffered water solution of trehalose, and is intended for the preservation (storage) of stem cells for a period necessary before their application to a patient, at a temperature of approximately 4 °C.

### BACKGROUND OF THE INVENTION

The utilisation of stem cells, and particularly mesenchymal stem cells (MSCs) is increasing because of their marked immunomodulatory, anti-inflammatory and antioxidative properties. Stem cells also produce various paracrine factors beneficial for the regeneration of affected tissue, while being able to differentiate into cells of the affected tissue. Due to these properties, they are utilised in modern medicine for the treatment of various diseases.

Before application of MSCs intended for therapeutic purposes, they must be characterised and tested properly. Most importantly it is necessary to confirm their safety (sterility tests, presence of endotoxins and Mycoplasma) and their identity (surface markers). MSCs must be transported from the place of production to the place of administration of the cells to the patient. All these procedures prolong the time for storing the cells after harvest (after finishing of their production) and increase the demands on the prolonged viability of the cells. As Sohn et al. (Cytotherapy, 2013) showed, the MSCs viability in a saline solution already dropped after two hours of cultivation.

For the cell storage, modified solutions, primarily developed for the transport of organs (US 7951590 B2, US 6045990 A, WO 2010064054 A1), are commonly applied.

The use of various solutions for the long-term preservation of mesenchymal stem cells has been described in the following publications; Ginis et al. (Tissue Eng Part C Methods, 2012) tested the preservation of adherent mesenchymal stem cells in Hypothermosol^{®} FRS solution (solution containing cations Na⁺, K⁺, Ca²⁺, Mg²⁺ and anions Cl⁻, H₂PO₄⁻, HCO₃⁻ and lactobionate, saccharose, mannitol, glucose, dextran-40, adenosine, glutathione, HEPES, Trolox) at 4 °C. The viability of this way preserved cells ranged from 70 % to 80 % after storing for 2 - 4 days. Chen et al. (Cell Transplant, 2013) tested the preservation of the cells in Plasmalyte solution (solution containing cations Na⁺, K⁺, Mg²⁺ and anions Cl⁻, CH₃COO⁻ and C₆H₁₁O₇⁻) containing human serum and dextrose. Corwin et al. (Cryobiology, 2014) tested other commercially available media and solutions, such as HBSS (Hank's Balanced Salt Solution) and ViaSpan^{®} (solution containing KH₂PO₄, MgSO₄, potassium lactobionate, raffinose, adenosine, glutathione, allopurinol, hydroxyethyl starch). The viability of MSCs after 48-hour storage at 4 °C was 45 %. The publication of Pogozhykh et al. (PLoS One, 2015) describes the preservation of approximately 50% MSCs viability after 24-hour storage of the cells at 4 °C in cultivation medium DMEM (Dulbecco's modified Eagle's medium) with 10% fetal bovine serum.

The described solutions contain a series of substances that should maintain the right pH, provide the optimal osmolarity of the solution, and protect the cells against intracellular oedema, shrinkage, free radicals, apoptosis and other negative effects of hypothermia during the preservation of cells.

Trehalose, a glucose disaccharide which is synthesised by lower organisms if they are exposed to stress, chill, high temperature or drying, is known as an anti-stress factor. It is frequently used in human medicine, for example to prevent water loss from the cells (dehydration), when the cells are exposed to excessive heat, cold or oxygen radicals, against the effects of hypoxia to anoxia as well as against hypoxia-reoxygenation injury. Due to its' unique properties, trehalose is used in food, pharmaceutical and cosmetic industries.

It is supposed, that trehalose prevents the denaturation and inactivation of proteins, and protects the lipid bi-layer of cell membranes against damage. Trehalose also likely serves as a source of carbon and glucose to provide cell functions, and also, as an absorber of free radicals, it provides protection against oxidative stress. Thus, it is in some cases used in solutions for the long-term preservation of tissues and cells under hypothermic conditions, in cryopreservation and lyophilisation (freeze drying).

Patent WO 2014208053 A1 describes the utilisation of various saline solutions supplemented with dextrans and trehalose, suitable for the transplantation of mammalian cells.

Trehalose is also a part of the solutions for the preservation and transplantation of organs protected by US patents 6365338 B1 and US 8512940 B2.

Patent application US 20150351380 A1 also describes the addition of trehalose into a solution for the preservation of cells for regenerative medicine.

Patent application US 20130260461 A1 describes a solution containing trehalose and stem cells that suppresses coagulation and improves the viability of these cells.

A paper by Di et al. (J Cell Physiol, 2012) shows that the presence of trehalose in a solution for the preservation of mesenchymal stem cells markedly improves their viability when being stored at a temperature of 4 °C, and the best results were achieved with a 40 mM concentration of trehalose.

Most of these solutions were developed originally for the transport of organs intended for transplantation, and they are not primarily intended for application to a patient. These solutions are mostly composed of a large number of inorganic salts, organic acids, saccharides, polysaccharides, antioxidants, buffer and chelating agents, stabilizers or antiapoptotic factors which do not need to be, in all cases, approved for human use in intravenous, intraarterial, intramuscular, intrathecal, subcutaneous, subconjunctival, local and other administration.

### SUMMARY OF THE INVENTION

The above drawbacks have been removed in the solution for the preservation, transport and application of stem cells for medical utilisation, which consists of a buffered water solution of trehalose. This solution contains, as necessary components, cations K⁺ in a quantity of 20 to 50 mmol/l, cations Na⁺ in a quantity of 20 to 80 mmol/l; anions Cl- in a quantity of 0.5 to 40 mmol/l, anions PO₄³⁻ and/or HPO₄²⁻ and/or H₂PO₄⁻ in a total quantity of 20 to 65 mmol/l, and trehalose in a quantity of 250 to 300 mmol/l. The concentration of these components has been selected within these limits in a way that the pH of the solution is from 7.1 to 7.6. In the simplest embodiment of the invention, the solution according to the invention does not have to contain any other intentionally added components. In any case, the optimum total osmolarity of the solution shall not exceed 400 mOsmol/l.

In another embodiment of the invention, the solution can also contain cations Mg²⁺ and/or Ca²⁺ in a total quantity of 0 to 2 mmol/1 and anions SO₄^{2~} and/or HSO₄⁻ in a total quantity of 0 to 2mmol/l. If there are cations Mg²⁺ and/or Ca²⁺ as intentionally added components, they are present in the solution in a total quantity of 0.1 to 2 mmol/l. If there are anions SO₄²⁻ and/or HSO₄⁻ as intentionally added components, they are present in the solution in a total quantity of 0.5 to 2 mmol/l.

In a preferred embodiment of the invention, the solution contains cations K⁺ in a quantity of 28 to 32 mmol/l, cations Na⁺ in a quantity of 20 to 50 mmol/l, cations Mg²⁺ and/or Ca²⁺ in a total quantity of 0.4 to 1.2 mmol/l, anions Cl⁻ in a quantity of 0.5 to 20 mmol/l, anions PO₄³⁻ and/or HPO₄²⁻ and/or H₂PO₄⁻ in a total quantity of 40 to 50 mmol/l, anions SO₄²⁻ and/or HSO₄⁻ in a total quantity of 0.9 to 1.2 mmol/l.

It is particularly advantageous when the water solution contains no other intentionally added components beside cations K⁺, cations Na⁺, cations Mg²⁺ and/or Ca²⁺, anions Cl-, anions PO₄³⁻ and/or HPO₄²⁻ and/or H₂PO₄⁻, anions SO₄²⁻ and/or HSO₄⁻ and trehalose. Of course, one cannot eliminate that there are traces of substances in the solution; their presence is caused by the production or otherwise accidental.

It is necessary to extend the shelf life of stem cells before administration to a patient to test their sterility, which is a necessary step before any administration of the cells to a patient. The extension of shelf life and preservation of the viability of the cells is also necessary for possible transport of the cells to a greater distance.

More specifically, the solution according to the invention is intended to store the cells at a temperature of -4 °C to 25 °C, advantageously 0 °C to 8 °C, and most frequently 4 °C to 6 °C. Adequate viability of the cells is kept for a period of at least 72 hours. The solution according to the invention consists of pharmaceutically acceptable compounds and it can be used directly for the application of the stem cells that are kept within.

According to this, the essence of the invention also lies in the use of the solution with formulation according to the invention for extending the viability of stem cells, for example at a temperature of 0 °C to 8 °C for a period up to 72 hours.

The solution, according to the invention, is intended particularly for the preservation of mesenchymal stem cells obtained from bone marrow, adipose tissue, peripheral blood or from extraembryonic tissues (placenta, umbilical cord, umbilical cord blood) or embryonic nerve, induced or limbal stem cells of mammals, including humans. It can also serve as preservation for stem cells attached to a biological scaffold.

The solution according to the invention, containing relevant stem cells, is intended for the treatment of inflammatory diseases, including post-traumatic inflammatory reactions after damage or injury, as well as for the treatment of degenerative and neurodegenerative diseases. The solution according to the invention containing relevant stem cells, can also be used for the treatment of trauma, developmental defects, healing of wounds and skin burns, for tissue replacement, for treatment of locomotion system diseases (tendons, joints, inflammatory diseases, arthritis, osteoarthritis), bone defects, diabetes, heart attacks, and cardiological and oncological diseases.

The stem cells, kept in the solution according to the invention, can be applied directly to a patient, without the necessity to wash-off this solution, and particularly in an intravenous, intraarterial, intramuscular, subcutaneous, subconjunctival or intrathecal way, can be applied either separately or with a scaffold.

Functional biomaterials can be the scaffolds for stem cells. They can be synthetic biocompatible polymers (polylactic acid PLA, polyglycolic acid PGA, polyethylenglycol PEG, polycaprolactone PCL, polyhydroxybutyrate PHB and other), natural polymers and polysaccharides (collagen type I, hyaluronic acid, fibrin, chitosan, cellulose, starch and other) or extracellular matrices (ECM) in the form of hydrogel, nanofibers or microfibers. The scaffold can be from biodegradable or non-degradable material.

In another embodiment, in the applications that expect the use of a scaffold, the cultivation processes of stem cells can be performed in the presence of these scaffolds and can be stored and applied together in the solution according to the invention.

Another option is that the stem cells can be kept in the solution according to the invention and mixed with a biocompatible scaffold before application to a patient, and administered to the patient together.

### BRIEF DESCRIPTION OF THE DRAWINGS

The attached figures show differentiated mesenchymal stem cells cultivated from bone marrow (BM-MSCs), after 72 hours of storage in the solution according to the invention at low temperature. The figures illustrate the ability of BM-MSCs to differentiate into adipocytes, chondrocytes and osteocytes.

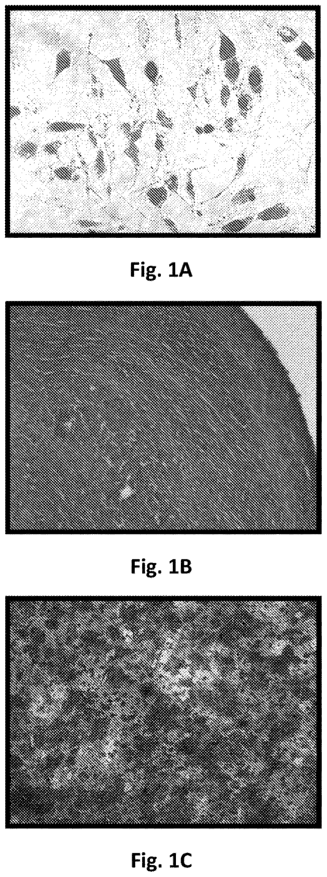

- Fig. 1A: shows adipogenic differentiation,
- Fig. 1B: shows chondrogenic differentiation, and
- Fig. 1C: shows osteogenic differentiation.

### EXAMPLES

### EXAMPLE 1: Isolation and culture of MSCs from bone marrow (BM-MSCs)

Bone marrow aspiration was performed by a trephine biopsy needle that is a component of the sampling set containing a saline solution with heparin and gentamicin. After the transportation of bone marrow for sterile processing, the sample was mixed with Gelofusin (infusion solution containing Na⁺ and Cl⁻ ions, and succinylated gelatin). Added Gelofusin corresponded to 25 % of total bone marrow volume. The mixture was left to sediment for 10 - 30 minutes followed by collection of the ring layer of mononuclear cells. The cellularity in the collected layer was evaluated by a hematology analyser. For primary culture establishment, the cell suspension (5 - 10 million nuclear cells) was then plated to culture flasks with a 75 cm² surface area in 10 ml of complete culture media (Alpha MEM Eagle medium containing 5 % human platelet lysate and gentamicin per flask).

The cells were kept at 37 ± 0.5 °C and in 5% CO₂ atmosphere. During cultivation, the culture medium was regularly changed with an optional rinse of 10 ml of PBS buffer per culture flask.

The cells were passaged according to their proliferation, i.e. adherent cells were detached enzymatically and transferred to a larger culture surface. This process involved culture inspection by light microscopy. When the cells reached 80-90% confluence, medium was aspired followed by rinsing with 10 ml of PBS and the addition of 1 ml of enzyme solution TrypLE Select CTS. The cells were incubated with the enzyme for approximately 4 min at 37 ± 0.5 °C.

The enzymatic reaction was terminated by the addition of 1 ml of 20% human serum albumin (HSA) and 6 ml of PBS, after the cells started to move in the liquid after gentle tapping of the flasks. The resulting suspension was gently agitated by repeated up and down pipetting, transferred to a centrifuge tube and centrifuged at 230 x g for 5 min.

The obtained cell pellet was resuspended in complete culture medium and the suspension was distributed into the desired number of flasks. The final cell suspension was achieved at the third passage. The cells were washed with PBS and centrifuged at 230 x g for 5 min.

To create the composition according to the invention, the pellet obtained as described above was resuspended in a solution described in Example 4A (250 mM trehalose which is further referred to as TR-Y-250), 4B (200 mM trehalose, referred to as TR-Y-200) and in Hypothermosol^{®} FRS (referred to as HTS).

The number of stem cells usually ranged from 5 × 10⁵ to 1 × 10⁷ MSCs/ml. Of note, 1 × 10⁶ MSCs/kg of patient weight can be administered intravenously.

### EXAMPLE 2: Isolation of adipose MSCs (AT-MSCs)

40 - 200 ml of lipoaspirate was collected from volunteers using standard tumescent liposuction. AT-MSCs were isolated from lipoaspirate by 60-min enzyme digestion using 0.3 U/mL of Collagenase NB 6 GMP grade.

Digested lipoaspirate was washed with PBS and centrifuged at 230 x g for 5 min at room temperature. The pellet was resuspended in the culture medium and seeded into culture flasks with a surface area of 75 cm². Further sample preparation was performed according to Example 1.

### EXAMPLE 3: Isolation of umbilical cord MSCs (WJ-MSCs)

Human umbilical cord was obtained from healthy newborns immediately after birth. Umbilical arteries and the vein were removed, and the umbilical cord tissue (Wharton's jelly, WJ) was cut into small pieces (approximately 1-2 mm³).

MSCs from umbilical tissue were isolated by the explant method or enzymatic digestion.

In the explant method, pieces of tissue were placed into culture flasks and were removed after 10 days. Outgrown cells attached in a monolayer were further cultured and treated according to Example 1.

The cell suspension obtained after enzymatic digestion was further processed and cultured according to Example 2.

### EXAMPLE 4: Options for solution preparation according to the invention

### EXAMPLE 4A: Preparation of the solution with 250 mM trehalose (TRY 250) according to the invention.

Weigh 0.408 g of KH₂PO₄ and 0.213 g of Na₂HPO₄, dissolve in 20 ml of distilled water; weigh 0.007 g of CaCl₂ × 2H₂O and dissolve in 10 ml of distilled water; weigh 0.012 g of MgSO₄ and dissolve in 10 ml of distilled water. Mix the obtained solutions and adjust the pH to 7.4. Then, dissolve 9.45 g of trehalose in the mixture and adjust the final volume with distilled water to 100 ml. The resulting osmolarity of the final solution is 358 mOsm.

### EXAMPLE 4B: Preparation of the solution with 200 mM trehalose (TRY 200) according to the invention

Weigh 0.408 g of KH₂PO₄ and 0.213 g of Na₂HPO₄, dissolve in 20 ml of distilled water; weigh 0.007 g of CaCl₂ × 2H₂O and dissolve in 10 ml of distilled water; weigh 0.012 g of MgSO₄ and dissolve in 10 ml of distilled water. Mix the obtained solutions and adjust the pH to 7.4. Then, dissolve 7.56 g of trehalose in the mixture and adjust the final volume with distilled water to 100 ml. The resulting osmolarity of the final solution is 308 mOsm.

### EXAMPLE 4C: Preparation of the solution with 250 mM trehalose according to the invention.

Weigh 0.816 g of KH₂PO₄ and 0.426 g of Na₂HPO₄ and dissolve in 20 ml of distilled water; weigh 0.014 g of CaCl₂ × 2H₂O and dissolve in 10 ml of distilled water; weigh 0.024 g of MgSO₄ and dissolve in 10 ml of distilled water. Mix the obtained solutions, adjust the pH to 7.4 adjust the final volume with distilled water to 100 ml (mixture 1).

Dissolve 37.8 g of trehalose in the distilled water and adjust the volume to 100 ml (mixture 2).

The final solution is formed by combining the three parts of mixture 2 with six parts of mixture 1 and three parts of distilled water. The adjustment of pH is not required. The resulting osmolarity of the final solution is 358 mOsm.

### EXAMPLE 4D: Preparation of the solution with 200 mM trehalose according to the invention.

Weigh 0.816 g of KH₂PO₄ and 0.426 g of Na₂HPO₄ and dissolve in 20 ml of distilled water; weigh 0.014 g of CaCl₂ × 2H₂O and dissolve in 10 ml of distilled water; weigh 0.024 g of MgSO₄ and dissolve in 10 ml of distilled water. Mix the obtained solutions, adjust the pH to 7.4 and adjust the final volume with distilled water to 100 ml (mixture 1).

Dissolve 37.8 g of trehalose in the distilled water and adjust the volume to 100 ml (mixture 2).

The final solution is formed by combining two parts of mixture 2 with five parts of mixture 1 and three parts of distilled water. The adjustment of pH is not required. The resulting osmolarity of the final solution is 308 mOsm.

### EXAMPLE 5: Viability of the BM-MSCs, AT-MSCs and WJ-MSCs after 48-72 hours of cold storage.

The viability of MSCs isolated from human bone marrow, adipose and umbilical tissue was evaluated after 48 and 72 hours of storage at a temperature of approximately 4 °C. BM-MSCs, AT-MSCs and WJ-MSCs were prepared according to Examples 1, 2 and 3, respectively. The MSCs were harvested and resuspended in the solution according to the invention as described in Example 1. After 48 or 72 hours, the viability of cells was determined by three independent methods.

Firstly, the suspension was stained with propidium iodide and the viability of cells was determined by flow cytometry. This dye binds to the cells with disrupted plasma membrane, while undamaged live cells remain unstained. Secondly, the same volume of cells that corresponds to the number of 100,000 fresh cells was seeded into 24- and 96-well tissue culture plates. After 24 hours, Alamar blue^{®} assay was performed in the 96-well plate. This assay evaluates the metabolic function of cultivated cells. The cells cultured in the 24-well plate were harvested and counted using a Bürker chamber. The results were compared with those obtained at time 0 (immediately after harvest, final passage). The Hypothermosol^{®} FRS (HTS) solution according to US 8642255 was used as the control preservation solution.

The propidium iodide staining reflects the viability of the individual cells in the suspension. Propidium iodide is a substance that does not pass through the membrane of viable cells. In damaged and non-viable cells, propidium iodide crosses the disintegrated plasma membrane and binds to the DNA. The viability of BM-MSCs assessed by propidium iodide staining immediately after the 48 hours of storage was above 80 % for all studied solutions (Table 1). After 72 hours of storage, the viability of cells in the TR-Y 250 and HTS solutions decreased by about 5 %, while in the TR-Y 200 solution, the viability was lowest at 73.4 ± 3.1 %. Finally, to assess the functionality of BM-MSCs after the cold storage, the cells were cultured overnight in the complete medium to allow their adherence to the surface. Adherence to culture plastic is one of the characteristics of viable stem cells. Adherent cells were harvested and their number was determined using the Bürker chamber.

The coefficient of cell adhesion was determined as the ratio between the number of harvested and plated (seeded) cells. After 48 hours of cold storage, the coefficient of cell adhesion ranged from 80 to 90 % and was highest in cells stored in the TR-Y 250 solution.

After 72 hours of storage, further decrease was observed, however the lowest values were obtained in cells stored in HTS solution (66 ± 4.4 %). The metabolic activity of adherent cells was also monitored by the Alamar blue^{®} assay.

The active ingredient of the Alamar blue^{®} is resazurin, a compound that penetrates all the cells. Viable, metabolically active cells can transform this blue compound into a bright red fluorescent dye resorufin. The fluorescence or absorbance level is directly proportional to the number of living cells and corresponds to the metabolic activity of the cells. For this assay, the cells were cultured overnight to ensure adherence, then a 10% Alamar blue^{®} solution was added to the complete culture medium and BM-MSCs were incubated for 4 hours at 37 °C. The fluorescence intensity was evaluated in a microplate reader using excitation and emission wavelengths of 535 nm and 550 nm, respectively. Metabolic activity of the adherent cells was presented as the ratio between the fluorescence values of BM-MSCs after 48 or 72 hours of storage and those of the respective cells before storage (after harvesting). In all studied solutions, the metabolic activity decrease dynamics was similar to the cell adhesion coefficient reduction dynamics of BM-MSCs.

The results of all BM-MSCs viability parameters (summarized in Table 1), show that the most suitable solution for the 48-72 hours of cold storage is the TR-Y 250 solution. The commercially available HTS solution provided the lowest cell viability after 48 hours of cold storage while after 72 hours, only one out of the three monitored parameters was better than TR-Y 200.

**Table 1 - Bone marrow MSCs**

| | Viability; propidium iodide staining, % of live (negative) cells | | Coefficient of cell adhesion; % of adherent cells related to 0 hours of storage | | Metabolic activity of adherent cells; Alamar blue^{®} staining; % related to 0 hours of storage | |
|---|---|---|---|---|---|---|
| | 48 hours | 72 hours | 48 hours | 72 hours | 48 hours | 72 hours |
| TR-Y250 | 88.3 ± 4.3 | 84.2 ± 0.8 | 90.1 ± 5.2 | 83.0 ± 4.5 | 87.4 ± 4.1 | 80.4 ± 2.8 |
| TR-Y 200 | 86.5 ± 3.2 | 73.4 ± 3.1 | 83.2 ± 3.3 | 76.0 ± 3.7 | 87.1 ± 3.8 | 73.0 ± 4.2 |
| HTS | 85.1 ± 1.6 | 80.9 ± 3.6 | 80.7 ± 2.1 | 66.0 ± 4.4 | 80.8 ± 1.7 | 72.2 ± 2.2 |

| | | | | | | |
|---|---|---|---|---|---|---|
| (TR-Y 250) solution according to the invention, containing 250 mM of trehalose; (TR-Y 200) solution according to the invention, containing 200 mM of trehalose; (HTS) Hypothermosol^{®} - FRS solution. | | | | | | |

The viability of AT-MSCs evaluated by propidium iodide staining after 48 hours of cold storage was approximately 95 % for all studied groups, and further decreased by 5 - 7 % after 72 hours (Table 2). However, the cell adhesion parameters were lower by 15 0 after 48 hours of storage as compared to the propidium iodide staining method. The lowest efficiency for cold storage for AT-MSCs was provided by the TR-Y 200 solution. The differences between TR-Y 250 and HTS were not significant after 48 hours of storage. However, TR-Y 250 showed significantly better results than HTS after 72 hours of cold storage, as for the coefficient of cell adhesion and their metabolic activity.

**Table 2 - Adipose tissue MSCs**

| | Viability; propidium iodide staining, % of live (negative) cells | | Coefficient of cell adhesion; % of adherent cells related to 0 hours of storage | | Metabolic activity of adherent cells; Alamar blue^{®} staining; % related to 0 hours of storage | |
|---|---|---|---|---|---|---|
| | 48 hours | 72 hours | 48 hours | 72 hours | 48 hours | 72 hours |
| TR-Y250 | 95.2 ± 5.5 | 90.1 ± 4.1 | 79.6 ± 3.7 | 67.4 ± 3.5 | 80.5 ± 3.8 | 65.3 ± 2.3 |
| TR-Y 200 | 94.5 ± 3.6 | 87.5 ± 4.2 | 77.3 ± 2.1 | 60.8 ± 5.5 | 75.2 ± 1.5 | 50.1 ± 5.3 |
| HTS | 96.1 ± 3.3 | 90.3 ± 2.7 | 79.3 ± 4.5 | 60.2 ± 5.3 | 78.7 ± 2.5 | 58.6 ± 4.7 |

| | | | | | | |
|---|---|---|---|---|---|---|
| (TR-Y 250) solution according to the invention, containing 250 mM of trehalose; (TR-Y 200) solution according to the invention, containing 200 mM of trehalose; (HTS) Hypothermosol^{®} - FRS solution. | | | | | | |

WJ-MSCs stored for 48 hours at 4 °C in the solution according to the invention containing 250 mM trehalose retained more than 90% viability (determined by the propidium iodide staining), while the viability of the cells stored in the HTS solution was only approximately 80 %. The results provided in Table 3 show that the cell adhesion coefficients were lower when compared to the propidium iodide staining, but the dynamics trend between individual solutions was maintained. The highest coefficient of WJ-MSCs adhesion after 72 hours of storage was achieved in the TRY 250 group (73.8 ± 4.3 %). Cells stored in the TR-Y 250 solution exhibited the highest viability, as assessed by all examined parameters. The lowest viability of WJ-MSCs assessed by all examined parameters was obtained after storage in the commercially available HTS solution.

**Table 3 - MSCs from umbilical cord tissue**

| | Viability; propidium iodide staining, % of live (negative) cells | | Coefficient of cell adhesion; % of adherent cells related to 0 hours of storage | | Metabolic activity of adherent cells; Alamar blue^{®} staining; % related to 0 hours of storage | |
|---|---|---|---|---|---|---|
| | 48 hours | 72 hours | 48 hours | 72 hours | 48 hours | 72 hours |
| TR-Y250 | 92.3 ± 3.2 | 85.3 ± 3.1 | 90.3 ± 2.8 | 73.8 ± 4.3 | 90.5 ± 3.3 | 81.0 ± 2.8 |
| TR-Y 200 | 88.7 ± 4.4 | 78.4 ± 2.7 | 78.4 ± 3.2 | 65.3 ± 5.2 | 87.6 ± 4.2 | 75.2 ± 5.1 |
| HTS | 83.4 ± 3.1 | 76.6 ± 2.1 | 77.2 ±4.1 | 55.4 ± 5.1 | 79.4 ± 2.5 | 60.6 ± 6.6 |

| | | | | | | |
|---|---|---|---|---|---|---|
| (TR-Y 250) solution according to the invention, containing 250 mM of trehalose; (TR-Y 200) solution according to the invention, containing 200 mM of trehalose; (HTS) Hypothermosol^{®}- FRS solution. | | | | | | |

These results show that cells stored in the TR-Y 250 and TR-Y 200 solutions for 48 to 72 hours under cold conditions maintain high viability.

### EXAMPLE 6: Phenotype of the BM-MSCs is maintained after 72 hours of cold storage - analysis of surface markers

Specific surface markers are one of the essential features characterising the identity of stem cells. For their detection, BM-MSCs were labelled with fluorescent conjugated antibodies.

After 72 hours of storage in TR-Y 250 according to the invention, BM-MSCs were labelled and analysed by flow cytometry at excitation wavelengths of 405 nm, 488 nm, and 633 nm. The following markers were tested: CD105, CD90, CD73, HLA-DR, CD45, CD34, CD14, CD19.

More than 90 % of the cells were positive for CD105, CD90 and CD73, while negativity (< 10 % of the cells) was observed for HLA-DR, CD45, CD34, CD14, and CD19. These results correspond with the proper phenotype of MSCs.

### EXAMPLE 7: Functional properties of BM-MSCs are maintained after 72 hours of cold storage

The ability of BM-MSCs to differentiate into adipocytes, osteocytes and chondrocytes after 72 hours of storage in the composition (solution TR-Y 250) according to the invention at about 4 °C was confirmed.

Cells stored for 72 hours in the TR-Y 250 solution under cold conditions were seeded onto plastic culture plates and cultured with appropriate differentiation media. After 21 days, the cells were fixed and stained with oil red, alcian blue and alizarin red for the evidence of adipogenic, chondrogenic and osteogenic differentiation, respectively (Fig. 1).

### EXAMPLE 8: Viability of WJ-MSCs attached to a biological scaffold after 48-72 hours of cold storage

WJ-MSCs from human umbilical cord tissue were prepared according to Example 3. After passaging according to Example 1, WJ-MSCs were seeded onto biological scaffold based on PCL nanofibers, at a density of 150,000 cells/cm². After two days of culture, the biological scaffold with attached cells was removed from the culture vial, rinsed with PBS and stored in the composition according to Example 4A at approximately 4 °C. The quantity of attached cells was determined by fluorescence staining.

Protocol using staining the microfilaments with phalloidin and cell nuclei with DAPI was applied. Microscopic evaluation of stained scaffolds showed that even after 72 hours of storage, a sufficient amount of living cells was attached with a 20% reduction of the viable cells number as compared to cells stained immediately after the scaffold removal from the culture medium. Furthermore, after 48 hours of storage, there was only a 15% decrease in the number of viable cells.

We assume that non-viable cells lost their adhesion ability while changing their shape from elongated to spherical, and were removed from the material during the staining procedure.

## Claims

1. A solution for a preservation, transport and application of stem cells obtained from bone marrow or adipose tissue or peripheral blood or from extraembryonic tissues or for stem cells attached to biological scaffold at a temperature of 0 °C to 8 °C for a period up to 72 hours, that consists of a buffer water solution of trehalose, **characterized by the fact** that the solution contains cations K⁺ in a quantity of 20 to 50 mmol/l, cations Na⁺ in a quantity of 20 to 80 mmol/l; anions Cl- in a quantity of 0.5 to 40 mmol/l, anions PO₄³⁻ and/or HPO₄²⁻ and/or H₂PO₄⁻ in a total quantity of 20 to 65 mmol/l, trehalose in a quantity of 250 to 300 mmol/l, and the solution, can also contain cations Mg²⁺ and/or Ca²⁺ in a total quantity of 0 to 2 mmol/l and anions SO₄²⁻ and/or HSO₄⁻ in a total quantity of 0 to 2 mmol/l, and pH of the solution is 7.1 to 7.6.

2. The solution according to claim 1, **characterized by the fact** that the solution contains cations Mg²⁺ and/or Ca²⁺ in a total quantity of 0.1 to 2 mmol/l.

3. The solution according to claims 1 or 2, **characterized by the fact** that the solution contains anions SO₄²⁻ and/or HSO₄⁻ in a total quantity of 0.5 to 2 mmol/l.

4. The solution according to claim 1, **characterized by the fact** that the solution contains cations K⁺ in a quantity of 28 to 32 mmol/l, cations Na⁺ in a quantity of 20 to 50 mmol/l, cations Mg²⁺ and/or Ca²⁺ in a total quantity of 0.4 to 1.2 mmol/l, anions Cl⁻ in a quantity of 0.5 to 20 mmol/l, anions PO₄³⁻ and/or HPO₄²⁻ and/or H₂PO₄⁻ in a total quantity of 40 to 50 mmol/l, anions SO₄²⁻ and/or HSO₄⁻ in a total quantity of 0.9 to 1.2 mmol/1.

5. The solution according to some of the claims 1 to 4, **characterized by the fact** that the solution consists of only cations K⁺, cations Na⁺, cations Mg²⁺ and/or Ca²⁺, anions Cl⁻, anions PO₄³⁻ and/or HPO₄²⁻ and/or H₂PO₄⁻, anions SO₄²⁻ and/or HSO₄⁻, of trehalose, water and possibly a trace quantity of accompanying dissolved substances.

## Patentansprüche

1. Mittel zur Aufbewahrung, zum Transport und zur Anwendung von Stammzellen, die aus Knochenmark oder Fettgewebe oder peripherem Blut oder aus extraembryonalen Geweben oder aus Stammzellen, die auf einem biologischen Träger fixiert sind, bei einer Temperatur von 0 °C bis 8 °C gewonnen wurden, bestehend aus einer gepufferten wässrigen Trehaloselösung, **dadurch gekennzeichnet, dass** die Lösung K⁺-Kationen in einer Menge von 20 bis 50 mmol/l, Na⁺-Kationen in einer Menge von 20 bis 80 mmol/l; Cl⁻-Anionen in einer Menge von 0,5 bis 40 mmol/l, PO₄³⁻-und/oder HPO₄²⁻- und/oder H2PO₄⁻-Anionen in einer Gesamtmenge von 20 bis 65 mmol/l, Trehalose in einer Menge von 250 bis 300 mmol/l enthält, wobei die Lösung ferner Mg²⁺- und/oder Ca²⁺-Kationen in einer Gesamtmenge von 0 bis 2 mmol/l und SO₄²⁻- und/oder HSO₄⁻-Anionen in einer Gesamtmenge von 0 bis 2 mmol/l enthalten kann, und wobei der pH-Wert der Lösung 7,1 bis 7,6 beträgt.

2. Mittel nach Anspruch 1, **dadurch gekennzeichnet, dass** die Lösung Mg²⁺- und/oder Ca²⁺-Kationen in einer Gesamtmenge von 0,1 bis 2 mmol/l enthält.

3. Mittel nach Anspruch 1 oder 2, **dadurch gekennzeichnet, dass** die Lösung SO₄²⁻- und/oder HSO₄⁻-Anionen in einer Gesamtmenge von 0,5 bis 2 mmol/l enthält.

4. Mittel nach Anspruch 1, **dadurch gekennzeichnet, dass** die Lösung K⁺-Kationen in einer Menge von 28 bis 32 mmol/l, Na⁺-Kationen in einer Menge von 20 bis 50 mmol/l, Mg²⁺- und/oder Ca²⁺-Kationen in einer Gesamtmenge von 0, 4 bis 1,2 mmol/l, Cl⁻-Anionen in einer Menge von 0,5 bis 20 mmol/l, PO₄³⁻- und/oder HPO₄²⁻- und/oder H₂PO₄⁻-Anionen in einer Gesamtmenge von 40 bis 50 mmol/l, SO₄²⁻- und/oder HSO₄⁻-Anionen in einer Gesamtmenge von 0,9 bis 1,2 mmol/l enthält.

5. Mittel nach einem der Ansprüche 1 bis 4, **dadurch gekennzeichnet, dass** die Lösung ausschließlich aus K⁺-Kationen, Na⁺-Kationen, Mg²⁺- und/oder Ca²⁺-Kationen, Cl⁻-Anionen, PO₄³⁻ - und/oder HPO₄²⁻- und/oder H₂PO₄⁻-Anionen, SO₄²⁻- und/oder HSO₄⁻-Anionen, Trehalose, Wasser und gegebenenfalls Spuren von Begleitlösungsmitteln besteht.

## Revendications

1. Moyen de conservation, de transport et d'application de cellules souches obtenu à partir de moelle osseuse ou de tissu adipeux ou de sang périphérique ou à partir de tissus extra-embryonnaires ou de cellules souches fixé sur un support biologique à une température de 0 °C à 8 °C, constitué d'une solution aqueuse tamponnée de tréhalose, **caractérisé en ce que** la solution contient des cations K⁺ en quantité de 20 à 50 mmol/l, des cations Na⁺ en quantité de 20 à 80 mmol/l ; des anions Cl⁻ en quantité de 0,5 à 40 mmol/l, des anions PO₄³⁻ et/ou HPO₄²⁻ et/ou H₂PO₄⁻ en quantité totale de 20 à 65 mmol/l, la tréhalose en quantité de 250 à 300 mmol/l, la solution pouvant en outre contenir des cations Mg²⁺ et/ou Ca²⁺ en une quantité totale de 0 à 2 mmol/l et des anions SO₄²⁻ et/ou HSO₄⁻ en une quantité totale de 0 à 2 mmol/l, le pH de la solution étant de 7,1 à 7,6.

2. Moyen selon la revendication 1 **caractérisé en ce que** la solution contient des cations Mg²⁺ et/ou Ca²⁺ en quantité totale de 0,1 à 2 mmol/l.

3. Moyen selon la revendication 1 ou 2 **caractérisé en ce que** la solution contient des anions SO₄²⁻ et/ou HSO₄⁻ en quantité totale de 0,5 à 2 mmol/l.

4. Moyen selon la revendication 1 **caractérisé en ce que** la solution contient des cations K⁺ en quantité de 28 à 32 mmol/l, des cations Na⁺ en quantité de 20 à 50 mmol/l, des cations Mg²⁺ et/ou Ca²⁺ en quantité totale de 0,4 à 1,2 mmol/l, des anions Cl⁻ en quantité de 0,5 à 20 mmol/l, des anions PO₄³⁻ et/ou des anions ou HPO₄²⁻ et/ou H₂PO₄⁻ en quantité totale de 40 à 50 mmol/l, des anions SO₄²⁻ et/ou HSO₄⁻ en quantité totale de 0,9 à 1,2 mmol/l.

5. Moyen selon l'une quelconque des revendications 1 à 4 **caractérisé en ce que** la solution est constituée exclusivement de cations K⁺, de cations Na⁺, de cations Mg²⁺ et/ou Ca²⁺, d'anions Cl⁻, d'anions PO₄³⁻ et/ou HPO₄²⁻ et/ou H₂PO₄⁻, d'anions SO₄²⁻ et/ou HSO₄⁻, de tréhalose, d'eau et éventuellement de traces de substances dissoutes d'accompagnement.
